(19) Europäisches Patentamt
European Patent Office
Office européen des brevets

(11) EP 3 443 973 B1

(12) **EUROPEAN PATENT SPECIFICATION**

(45) Date of publication and mention of the grant of the patent:
**12.02.2025 Bulletin 2025/07**

(21) Application number: **17782172.5**

(22) Date of filing: **14.03.2017**

(51) International Patent Classification (IPC):
***A61K 36/195** (2006.01)* ***A61K 31/122** (2006.01)*
***A61P 25/28** (2006.01)* ***A61P 25/00** (2006.01)*

(52) Cooperative Patent Classification (CPC):
**A61P 25/00; A61K 31/122; A61K 36/195**

(86) International application number:
**PCT/JP2017/010083**

(87) International publication number:
**WO 2017/179354 (19.10.2017 Gazette 2017/42)**

(54) **COMPOSITION FOR IMPROVING RECOGNITION FUNCTIONS**

ZUSAMMENSETZUNG ZUR VERBESSERUNG VON ERKENNUNGSFUNKTIONEN

COMPOSITION POUR AMÉLIORER LES FONCTIONS DE RECONNAISSANCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2016 JP 2016079304**

(43) Date of publication of application:
**20.02.2019 Bulletin 2019/08**

(73) Proprietor: **Kirin Holdings Kabushiki Kaisha**
**Tokyo 164-0001 (JP)**

(72) Inventors:
- **KANAYAMA, Masaya**
  **Tokyo 164-0001 (JP)**
- **ANO, Yasuhisa**
  **Tokyo 164-0001 (JP)**
- **AYABE, Tatsuhiro**
  **Tokyo 164-0001 (JP)**
- **TANIGUCHI, Yoshimasa**
  **Tokyo 164-0001 (JP)**
- **MATSUKURA, Yasuko**
  **Tokyo 164-0001 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 653 165** **EP-A1- 2 865 744**
**EP-A1- 2 865 745** **WO-A1-2012/081675**
**WO-A1-2013/191264** **JP-A- 2015 224 193**
**JP-A- 2015 224 194** **JP-A- S5 070 512**

- **DATABASE WPI Week 201536, Derwent World Patents Index; AN 2015-30283B, XP002794964**
- **DATABASE WPI Week 201612, Derwent World Patents Index; AN 2016-04468Y, XP002794965**
- **NORIO SASAOKA ET AL.: "Oral Administration of Hop Flower Extracts Mitigates Alzheimer Phenotypes in Mice", BIO IND., vol. 31, no. 12, 2014, pages 34 - 39, XP9513323, ISSN: 0910-6545**
- **LU YH. ET AL.: "DNA strand-scission by phloroglucinols and lignans from heartwood of Garcinia subelliptica Merr And Justicia plants", PHYTOCHEMISTRY, vol. 69, no. 1, 2008, pages 225 - 233, XP022392524, ISSN: 0031-9422**
- **TOBE H ET AL.: "The neuroprotective effect of isohumulone on swine brain cells ", BULLETIN OF KOCHI NATIONAL COLLEGE OF TECHNOLOGY, 1 January 2011 (2011-01-01), pages 59 - 63, XP055579661, ISSN: 0454-1170**
- **ANO Y ET AL.: "Iso-alpha-acids, bitter components of beer, prevent inflammation and cognitive decline induced in a mouse model of Altzheimer's disease", J BIOL CHEM., vol. 292, no. 9, 3 March 2017 (2017-03-03), pages 3720 - 3728, XP055429585, ISSN: 1067-8816**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 443 973 B1

Remarks:

The file contains technical information submitted after the application was filed and not included in this specification

## Description

CROSS-REFERENCE TO RELATED APPLICATION

This application enjoys the benefit of priority of the prior Japanese Patent Application No. 2016-79304 (filed on April 12, 2016).

TECHNICAL FIELD

as claimed for use in a method according to the claims. The present invention relates to a composition

BACKGROUND ART

Cognitive functions are required to be maintained, enhanced and improved in all generations from younger to older. It is important not only for students and adults who study for examinations such as entrance examinations and qualification tests, but also for people who conduct daily business and private lives, to maintain, enhance, and improve memory and learning abilities. Also, a weak memory and poor concentration may affect quality of life in elderly people. Thus, it is required to prevent cognitive decline and to maintain, enhance, and improve cognitive functions.

Furthermore, in elderly people, the number of cases of age-related mental disorders that cause cognitive decline is rising with the increasing number of elderly people, which is a growing social problem. Not only dementia represented by Alzheimer's disease but also mental disorders such as depression and delirium are reported as diseases that cause cognitive decline (Non-Patent Document 1).

Meanwhile, hops are responsible for bitterness in beer and have a long history of use in folk medicine and have various health benefits such as sedative effect and antiindigestion effect. Foods and beverages hop-extract added beyond a certain concentration have a distinct and strong bitter taste, so it may damage the palatability of the food and beverage. However, it is reported that oxidation-treatemented hops can be inhibited with the bitter taste attributable to hops,and the oxidation-treated hops is maintained its lipid metabolism improving ability (Patent Document 1). However, there is so far no report describing the relationship between hop-derived components or oxidation products thereof and cognitive functions.

REFERENCE LIST

[Patent Document]

Patent Document 1: WO2012/081675

[Non-Patent Document]

Non-Patent Document 1: Toshihisa Tanaka, and Masatoshi Takeda. (2011) Nippon-Rinsho, Supplementary Volume, pp. 52-56.

SUMMARY OF THE INVENTION

In this invention, the inventors found that a hop oxidation-reaction product was effective in maintenance, enhancement, improvement, etc. of a cognitive function. The present invention is based on the finding.

In other words, an object of the present invention is to provide a composition that is effective in maintenance, enhancement, improvement, etc of a cognitive function.

According to the present invention, the following inventions are provided.

1. A composition for use in a method of maintaining, enhancing, and/or improving a cognitive function, comprising a hop oxidation-reaction product, wherein oxidation is performed by heating hop in the air at a temperature between 60 °C and 100 °C, wherein said hop contains lupulin glands, and wherein said hop oxidation-reaction product is provided as an extract in water, wherein the extraction temperature is 60 °C or lower, wherein the hop oxidation-reaction product has a ratio of the total amount of tricyclooxyisohumulone A and tricyclooxyisocohumulone A to the total amount of scorpio-humulinol A and scorpio-cohumulinol A from 2 to 20.

2. The composition for use according to item 1, wherein the cognitive function is memory function.

3. The composition for use according to item 1, wherein the cognitive function is attention or concentration function.

4. The composition for use according to any one of items 1 to 3, which is a food composition.

5. The composition for use according to any one of items 1 to 4, which is in a single unit package suitable for a single ingestion.

6. The composition for use according to any one of items 1-5, which comprises the hop oxidation-reaction product in an amount of 8 to 1700 mg on the dry-mass basis for a single ingestion.

7. The composition for use according to any one of items 1 to 6, for use in the treatment, prevention, or amelioration of memory impairment, disorientation, and/or cognitive impairment.

8. Use of a hop oxidation-reaction product for the manufacture of a composition or food product for maintaining, enhancing, and/or improving a cognitive function, or for the manufacture of an agent for maintaining a cognitive function, an agent for enhancing a cognitive function, and an agent for improving a cognitive function, wherein oxidation is performed by heating hop in the air at a temperature between 60 °C and 100 °C, wherein said hop contains lupulin glands, and wherein said hop oxidation-reaction product is provided as an extract in water, wherein the extraction temperature is 60 °C or lower.

The present invention provides a composition as claimed and for the claimed use containing a hop oxidation-reaction product that can exert functions such as maintenance, enhancement, and improvement of a cognitive function. A hop-derived component which has been long ingested as a food by human is used for the hop oxidation-reaction product and, therefore, the present invention is advantageous in that the composition according to the present invention can be used as a functional material that is safe for mammals including human.

BRIEF DESCRIPTION OF THE DRAWINGS

FIG. 1 depicts the result of HPLC analysis (HPLC chromatogram) of a hop oxidation-reaction product in Reference Example 1.
FIG. 2 depicts the test schedule for humans in Example 2.
FIG. 3 depicts an exemplary questionnaire for the Letter-Number Sequencing Test in Example 2.
FIG. 4 depicts a graph showing the result of the Trail Making Test in Example 2. A single asterisk (*) indicates a probability of <0.05 (t-test).
FIG. 5 depicts a graph showing the result of the Pattern Recognition Memory test in Example 2. A single asterisk (*) indicates a probability of <0.05 (t-test).
FIG. 6 depicts a graph showing the result of the Spatial Working Memory test (regarding "within errors") in Example 2. Single asterisks (*) indicate a probability of <0.05 (t-test).
FIG. 7 depicts a graph showing the result of the Spatial Working Memory test (regarding "between errors") in Example 2. A double asterisk (**) indicates a probability of <0.01 (*t*-test).
FIG. 8 depicts a graph showing the result of the Paired Associates Learning test in Example 2. A single asterisk (*) indicates a probability of <0.05 (*t*-test).
FIG. 9 depicts a graph showing the result of the Letter-Number Sequencing Test in Example 2. A single asterisk (*) indicates a probability of <0.05 (*t*-test).
FIGS. 10A and 10B depict the scheme of a Y-maze test and the schematic view of a Y-shaped maze, respectively.
FIG. 11 depicts a graph showing the total entry numbers (arm entry) in mice of Example 3.
FIG. 12 depicts a graph showing the spontaneous alternation rates in mice of Example 3.
FIG. 13 depicts the scheme of a novel object recognition test for mice in Example 4.
FIG. 14 depicts the ratios of time spent exploring a novel object to time spent exploring a familiar object (the discrimination index) in mice of Example 4.
FIG. 15 depicts the ratios of time spent for the exploration of a novel object in mice of Example 4.
FIG. 16 depicts a graph showing the total entry numbers (arm entry) in mice of Example 5.
FIG. 17 depicts a graph showing the spontaneous alternation rates in mice of Example 5.
FIG. 18 depicts the ratios of time spent exploring a novel object to time spent exploring a familiar object (the discrimination index) in mice of Example 5.
FIG. 19 depicts the ratios of time spent for the exploration of a novel object in mice of Example 5.

DETAILED DESCRIPTION OF THE INVENTION

Hop Oxidation-Reaction Products

In the present invention, hop oxidation-reaction products represent products obtained by subjecting hops or hop products (such as hop pellets and extracts) to oxidation. A hop oxidation-reaction product can be obtained by, for example, bringing hops into contact with oxygen in the air and thereby oxidizing the hops.

Hop oxidation-reaction products can be produced by oxidizing hops according to, for example, the method described in Patent Document 1. Oxidation is preferably performed by heating hops in the air. The heating temperature is not particularly limited, but the preferable upper limit is 100°C, and the more preferable upper limit is 80°C. A heating temperature of not higher than 100°C is advantageous for progression of oxidization in preference to isomerization. Moreover, the preferable lower limit of heating temperature is 60°C. A heating temperature of not lower than 60°C is advantageous for progression of oxidation in an efficient manner. Moreover, the reaction period is also not particularly limited, but can be appropriately determined depending on the variety of hop and the reaction temperature. For example, when the reaction temperature is at 60°C, a reaction period of 48-120 hours is preferred; when the reaction temperature is at 80°C, a reaction period of 8-24 hours is preferred. The form of hops to be subjected to oxidation is not particularly limited as long as they can be brought into contact with oxygen in the air, but processing hops into a powdery form can preferably shorten the required reaction time.

In the present invention, hops may have any form as long as they contain lupulin glands, and hops such as harvested hops before drying, harvested and dried hops, compressed hops, ground hops, or hops processed into a pellet form may be used and hops in a pellet form is preferably used. Commercially available hop pellets may be used and examples of the commercially available hop pellets include hop strobiles compressed into a pellet form (Type 90 pellet), pellets in which lupulin glands have been selectively concentrated (Type 45 pellet), or hop pellets subjected to isomerization treatment (for example, Isomerized Pellets (Hopsteiner Trading Co., Ltd)).

Hop extract oxidation-reaction products generated by subjecting hop extracts to oxidation may be provided as the hop oxidation-reaction products in the present invention. Hop extract oxidation-reaction products can be produced by oxidizing hop extracts according to, for example, the method described in Patent Document 1.

Hop contains acidic resin components such as α-acids (humulones), β-acids (luplones) and iso-α-acids (isohumulones). In the present invention, "humulones" is used to refer inclusively to humulone, adhumulone, cohumulone, posthumulone, and prehumulone. Moreover, in the present invention, "luplones" is used to refer inclusively to lupulone, adlupulone, colupulone, postlupulone, and prelupulone. Furthermore, in the present invention, "isohumulones" is used to refer inclusively to isohumulone, isoadhumulone, isocohumulone, isoposthumulone, isoprehumulone, Rhoisohumulone, Rho-isoadhumulone, Rho-isocohumulone, Rho-isoposthumulone, Rhoisoprehumulone, tetrahydroisohumulone, tetrahydroisoadhumulone, tetrahydroisocohumulone, tetrahydroisoprehumulone, tetrahydroisoposthumulone, hexahydroisohumulone, hexahydroisoadhumulone, hexahydroisocohumulone, hexahydroisoposthumulone, and hexahydroisoprehumulone. In addition, isohumulones include *cis*- and *trans*-stereoisomers and "isohumulones" is used to refer inclusively to both stereoisomers, unless otherwise specifically stated.

Subjecting hops to oxidation decreases the contents of α-acids, β-acids and iso-α-acids thereof and increases the contents of components other than those acids thereof. Examples of such a "hop oxidation-reaction product" include, among hop oxidation-reaction products, a hop oxidation-reaction product showing peaks of α-acids, β-acids and iso-α-acids at an area ratio of 20% or lower, preferably 10% or lower, of the total HPLC peaks, in cases where HPLC analysis similar to that in Example 1 is performed.

Other components, in addition to α-acids, β-acids and iso-α-acids, contained in an oxidation product according to the present invention can be readily detected by well-known analytical measures such as HPLC. For example, a hop oxidation-reaction product prepared by a procedure similar to that described in Example 1 of Patent Document 1 contains other components in addition to α-acids, β-acids and iso-α-acids and peaks corresponding to those other components (also referred to collectively as "S-fraction (S-Fr)" in this specification) can exhibit bioactivities. Peaks within the ranges indicated by arrows in FIG. 1A for Example 1 of Patent Document 1 (excluding the peaks of α-acids and β-acids) correspond to the S-fraction.

A HPLC analysis of a hop oxidation-reaction product prepared under the same conditions as those in Patent Document 1 and the result of the analysis (HPLC chromatogram) are as demonstrated in Reference Example 1 and FIG. 1. Peaks within the ranges indicated by Arrows A1 and A2 (excluding the peaks of α-acids and β-acids) correspond to the S-fraction. In FIG. 1, the area value of peaks within the ranges indicated by Arrows A1 and A2 represents the sum of the area values of peaks within the range A1, which corresponds to the range of retention time from 3 minutes to 25 minutes, and the area values of peaks within the range A2 (excluding the peaks of α-acids and β-acids), which corresponds to the range of retention time from 32 minutes to 39 minutes. As used herein, the phrase "to a retention time of 25 minutes" in the range A1 means "to a time point when a peak identified as corresponding to *trans*-isocohumulone appears". Moreover, characteristic peaks were observed around a retention time of 9.7 minutes, a retention time of 11.8 minutes and a retention time of

12.3 minutes within the range indicated by A1 in FIG. 1. Furthermore, shoulder peaks were observed within the range indicated by A2 in FIG. 1 and the starting point was around a retention time of 32 minutes, the peak top points (excluding the peaks of α-acids and β-acids) were within the range of retention time from around 35 minutes to around 36 minutes, and the ending point was around a retention time of 39 minutes.

The hop oxidation-reaction product preferably contains oxidation products of α-acids, those of iso-α-acids, and those of β-acids and contains, for example, "tricyclooxyisohumulones" as such oxidation products. As used herein, the term "tricyclooxyisohumulones" refers to a group of compounds including tricyclooxyisocohumulone A (TCOIcoH A, see the following formula 1; IUPAC name: (3 aS, 5 aS,7 S, 8aS)-3,3 a-dihydroxy-7-(1-hydroxy-1-methylethyl)-6,6-dimethyl-2-(2-methylpropanoyl)-5a,6,7,8-tetrahydro-3aH,5H-cyclopenta[c]pentalene-1,4-dione), tricyclooxyisohumulone A (TCOIH A, see the following formula 2; IUPAC name: (3 aS, 5 aS,7 S, 8aS)-3,3 a-dihydroxy-7-(1-hydroxy-1-methylethyl)-6,6-dimethyl-2-(3-methylbutyryl)-Sa,6,7,8-tetrahydro-3aH,5H-cyclopenta[c]pentalene-1,4-dione), and tricyclooxyisoadhumulone A (TCOIadH A, see the following formula 3; IUPAC name: (3 aS, 5 aS,7 S, 8aS)-3,3 a-dihydroxy-7-(1-hydroxy-1-methylethyl)-6,6-dimethyl-2-(2-methylbutanoyl)-5a,6,7,8-tetrahydro-3aH,5H-cyclopenta[c]pentalene-1,4-dione). In this specification, TCOIcoH A, TCOIH A and TCOIadH A may be hereinafter referred to collectively as TCOIHs A. The content of TCOIHs A is measured by a method as described in Example 1 below.

Formula 1

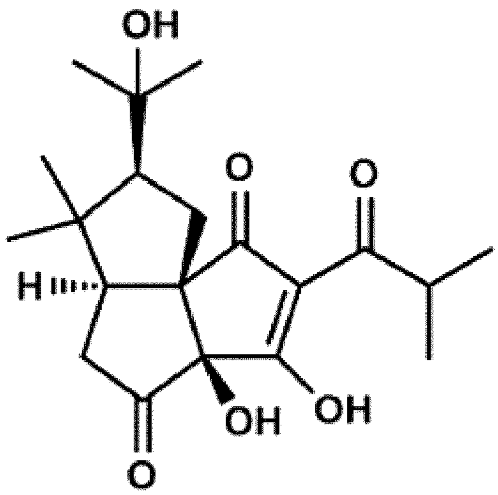

Formula 2

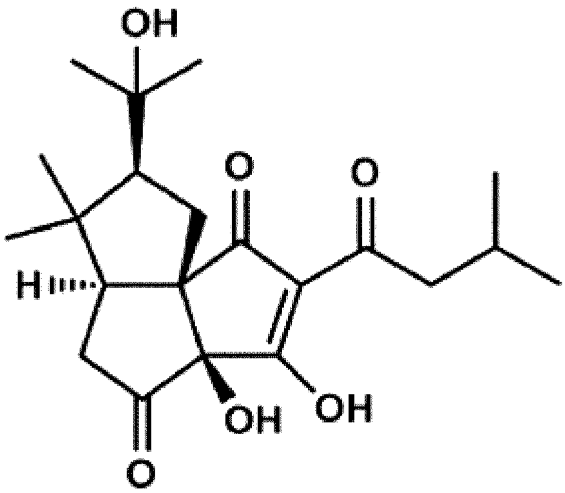

Formula 3

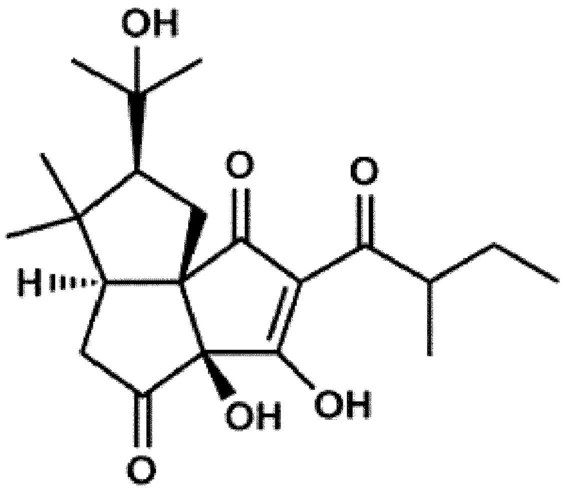

Oxidation products other than "tricyclooxyisohumulones" contained in the hop oxidation-reaction product (preferably the S-fraction) include scorpio-humulinol A and scorpio-cohumulinol A.

Hop oxidation-reaction products may be provided as an extract in an aqueous medium. The aqueous medium is not particularly limited as long as it is commonly used for manufacturing a food, but the aqueous medium is preferably water or ethanol and more preferably water. Moreover, the extraction temperature is not particularly limited, but is preferably at 60°C or lower and is more preferably in the range of 50-60°C in terms of extraction efficiency.

The hop oxidation-reaction product used in the present invention can be characterized by the ratio of the total amount of tricyclooxyisohumulone A and tricyclooxyisocohumulone A to the total amount of scorpio-humulinol A and scorpio-cohumulinol A (on the dry-mass basis), and an extract of the hop oxidation-reaction product in an aqueous medium with the ratio ranging, for example, from 1 to 30, preferably from 2 to 20, can be used.

Also, the hop oxidation-reaction product used in the present invention can be characterized by the content ratio of TCOIHs contained in the S-fraction (on the dry-mass basis), and a hop oxidation-reaction product with the content ranging, for example, from 5 to 15% by mass, preferably from 5 to 12% by mass, can be used. In cases where the S-fraction of a hop oxidation-reaction product comprises mainly the components in the fraction indicated by Arrow A1 in FIG. 1, the content of TCOIHs can be measured by a measurement method similar to that for matured hop bitter acids as described in Biosci. Biotechnol. Biochem., 2015 (79): 1684-1694.

The degree Brix of an extract of the hop oxidation-reaction product in an aqueous medium is not particularly limited, but it is, for example, not higher than 3 and preferably in the range of 1.5 to 3. Insoluble components in the extract of the hop oxidation-reaction product in an aqueous medium may be removed, for example, by decantation or with filter paper. The extract of the hop oxidation-reaction product in an aqueous medium may also be treated with activated charcoal.

In addition to the hop oxidation-reaction product, one, two, or more other components intended for the maintenance, enhancement, and/or improvement of a cognitive function may be contained in a composition according to the present invention. Examples of the other component intended for the maintenance, enhancement, and/or improvement of a cognitive function include ω-3 fatty acids such as eicosapentaenoic acid (EPA) and docosahexaenoic acid (DHA); polyphenols such as ginkgo leaf extract, resveratrol, and curcumin; lecithin, isohumulone, peptides, and vitamins that prevent abnormal homocysteine metabolism, which is a risk factor for Alzheimer's disease.

Uses

As shown in Examples below, the hop oxidation-reaction product (preferably, the S-fraction) has effects to maintain, enhance, and improve cognitive functions including memory function. Thus, the hop oxidation-reaction product can be used in a method of maintaining, enhancing, and improving a cognitive function, as well as can be used as an active ingredient in compositions for maintaining, enhancing, and/or improving a cognitive function. Also, the hop oxidation-reaction product can be used as an active ingredient in an agent for maintaining, an agent for enhancing, and an agent for improving a cognitive function. In the present invention, the term "cognitive function" is used to refer inclusively to memory function and attention or concentration function.

In the present invention, the term "memory function" refers to a function including spatial cognitive function, operation memory function, working memory function, episodic memory function, visual memory function, and learning function. Working memory includes language working memory, operational working memory and spatial working memory.

In the present invention, the "maintenance of memory function" includes, for example, preventing reduction in

memory function. Moreover, the "enhancement of memory function" includes, for example, achieving a higher level of memory function than ever before, and promoting the establishment of medium- and long-term memory and thereby promoting the development of the brain. Furthermore, the "improvement of memory function" includes, for example, recovering the once lowered memory function, and recovering symptoms that have signs of decline. Examples of the maintenance, enhancement, and/or improvement of memory function include enhancing memory function, and suppressing reduction in memory function.

In the present invention, the "maintenance of attention or concentration function" includes, for example, suppressing reduction in attention or concentration associated with aging in, for example, elderly people. Moreover, the "enhancement of attention or concentration function" includes, for example, transiently enhancing attention or concentration function, and promoting maintenance and enhancement of medium- and long-term attention or concentration function. Furthermore, the "improvement of attention or concentration function" includes, for example, recovering the once lowered attention or concentration function due to aging and others, and recovering seemingly reduced attention or concentration function. Examples of the maintenance, enhancement, and/or improvement of attention or concentration function include enhancing attention or concentration function, and suppressing reduction in attention or concentration function.

The above-described compositions for use in a method of maintaining, enhancing, and improving a cognitive function according to the present invention can be performed by feeding or administering an effective amount of the hop oxidation-reaction product to a human or a non-human animal. In other words, the present invention provides compositions for use in a method of maintaining, enhancing, and improving a cognitive function, which method comprises feeding or administering an effective amount of the hop oxidation-reaction product to a subject in need thereof. The fed or administered subject is a mammal, including a human, and is preferably a human.

The present invention also provides use of the hop oxidation-reaction product for the manufacture of a composition and a food product for maintaining, enhancing, and/or improving a cognitive function as claimed. The present invention further provides use of the hop oxidation-reaction product for the manufacture of an agent for maintaining, an agent for enhancing, and an agent for improving a cognitive function as claimed. The present invention still further provides use of the hop oxidation-reaction product and of a composition containing the same as an agent for maintaining, an agent for enhancing, or an agent for improving a cognitive function as claimed. The present invention still further provides a composition as claimed comprising a hop oxidation-reaction product for use in the maintenance, enhancement, and/or improvement of a cognitive function.

The use of the hop oxidation-reaction product in the present invention may be use in human and non-human animals, and is intended for both therapeutic use and non-therapeutic use. In this specification, the term "non-therapeutic" means excluding the act of performing surgery on, treating, or diagnosing a human (i.e., medical practices on humans) and specifically means excluding a procedure in which a physician or an individual who is directed by a physician performs surgery on, treats, or diagnoses a human.

Moreover, in the present invention, the hop oxidation-reaction product can be used to treat, prevent, or ameliorate diseases and symptoms which are effectively treated, prevented, or ameliorated by maintenance, enhancement, and/or improvement of a cognitive function.

Examples of the diseases and symptoms which are effectively treated, prevented, or ameliorated by maintenance, enhancement, and/or improvement of a cognitive function include diseases and symptoms which are effectively treated, prevented, or ameliorated by maintenance, enhancement, and/or improvement of memory function and/or attention or concentration, more particularly memory impairment (a condition causing difficulty in recollection of memory and difficulty in acquisition and retention of new information), disorientation (impaired awareness regarding time, place, or personal identity), and cognitive impairment (impaired calculation ability, impairment of judgment, aphasia, agnosia, apraxia, executive dysfunction). Thus, the hop oxidation-reaction product can be used as a therapeutic agent, a prophylactic agent, and an ameliorative agent for those diseases and symptoms, as well as can be used in a method of treating, a method of preventing, and a method of ameliorating those diseases and symptoms. When the therapeutic method, the prophylactic method and the ameliorative method according to the present invention are implemented, these methods can be performed by administering an effective amount of the hop oxidation-reaction product to a mammal, including a human, in need of treatment, prevention, or amelioration. In other words, the present invention provides compositions as claimed for use in a method of treating, a method of preventing, and a method of ameliorating diseases and symptoms which are effectively treated, prevented, or ameliorated by maintenance, enhancement, and/or improvement of a cognitive function, which methods comprise feeding or administering an effective amount of the hop oxidation-reaction product to a subject in need thereof. The fed or administered subject is a mammal, including a human, and is preferably a human.

The present invention also provides use of the hop oxidation-reaction product for the manufacture of a composition for treating, a composition for preventing, and a composition for ameliorating diseases and symptoms which are effectively treated, prevented, or ameliorated by maintenance, enhancement, and/or improvement of a cognitive function. These compositions are preferably pharmaceutical compositions. The present invention further provides use of

the hop oxidation-reaction product for the manufacture of an agent for treating, an agent for preventing, and an agent for ameliorating diseases and symptoms which are effectively treated, prevented, or ameliorated by maintenance, enhancement, and/or improvement of a cognitive function. Furthermore disclosed herein is the use of the hop oxidation-reaction product and of a composition comprising the same as an agent for treating, an agent for preventing, and an agent for ameliorating diseases and symptoms which are effectively treated, prevented, or ameliorated by maintenance, enhancement, and/or improvement of a cognitive function. The present invention still further provides a hop oxidationreaction product as claimed and a composition comprising the same for use in the treatment, prevention, and amelioration of diseases and symptoms which are effectively treated, prevented, or ameliorated by maintenance, enhancement, and/or improvement of a cognitive function.

The compositions for maintaining, enhancing, and/or improving a cognitive function according to the present invention, and the agents for maintaining, enhancing, and/or improving a cognitive function according to the present invention, and the therapeutic agent, the prophylactic agent and the ameliorative agent according to the present invention can be provided in the form of, for example, a medicament, a quasidrug, a food product, and a feed product (including pet food), and can be achieved as described below. Moreover, the method of maintaining, enhancing, and improving a cognitive function according to the present invention and the therapeutic method, the prophylactic method and the ameliorative method according to the claimed medical use can be implemented as described below. Furthermore, the uses according to the present invention can also be implemented as described below.

The composition, the agent, and the hop oxidation-reaction product according to the present invention, each of which is an active ingredient of the present invention, can be administered orally to human and non-human animals. Oral drugs include granules, powders, tablets (including sugar-coated tablets), pills, capsules, syrups, emulsions, and suspensions. These formulations can be formulated with pharmaceutically acceptable carriers, according to procedures commonly used in the art. Examples of the pharmaceutically acceptable carriers include, for example, excipients, binders, diluents, additives, flavoring agents, buffers, thickeners, coloring agents, stabilizing agents, emulsifiers, dispersing agents, suspending agents, and preservatives.

When the hop oxidation-reaction product, which is an active ingredient of the present invention, is provided as a food product, the hop oxidation-reaction product may be directly provided as a food product or be provided in a mixed form with another food. The thus provided food product is a food product containing an effective amount of the hop oxidation-reaction product. In this specification, the phrase "containing an effective amount" of the hop oxidation-reaction product refers to the content of the hop oxidation-reaction product (preferably, the S-fraction) in each food product which allows ingestion of the hop oxidation-reaction product in an amount within the range described below when the food product is taken in such an amount that the food product is usually eaten by individuals. Moreover, the term "food product" is used to refer inclusively to health foods, functional foods, food supplements, dietary supplements, health-promoting foods (for example, foods for specified health uses, functional nutritional foods, foods with function claims), and foods for special dietary use (for example, foods for infants, foods for pregnant women, foods for diseased people).

The composition, the agent, and the hop oxidation-reaction product according to the present invention have effects to maintain, enhance, and/or improve cognitive functions and thus may be provided in a mixed form with a daily ingested food, particularly with a food ingested as a dietary supplement. In this case, a predetermined amount of the composition, the agent, or the hop oxidation-reaction product according to the present invention may be provided in a single unit package for a single ingestion. Examples of the single unit package for a single ingestion includes packages configured to define a specific amount of a substance, such as carton, packaging container, can, and bottle. To better elicit the various effects of the composition, the agent, and the hop oxidation-reaction product according to the present invention, the amount of each of them to be ingested in a single meal may be determined depending on the amount of the hop oxidation-reaction product ingested in a single meal as described below. The food product of the present invention may be provided in a packaging container with a label for information on the ingested amount, or provided together with a document with the information.

The food product of the present invention may be labeled as having effects to maintain, enhance, and/or improve cognitive functions. In this case, the label for the food product of the present invention may contain some or all of the following claims for users' easy understanding. Of course, in the present invention, the phrase "maintenance, enhancement, and/or improvement of a cognitive function" is used to refer inclusively the following indications: (The food product of the present invention is intended for)

- retaining, boosting, enhancing, improving, increasing, supporting, and maintaining memory;
- retaining, boosting, enhancing, improving, increasing, supporting, and maintaining cognition;
- retaining, boosting, enhancing, improving, increasing, supporting, and maintaining attention;
- retaining, boosting, enhancing, improving, increasing, supporting, and maintaining concentration;
- helping maintenance of memory and preventing reduction in memory;
- preventing carelessness and forgetfulness;
- promoting the establishment of memory and increasing the accuracy of memory; and

- suppressing reduction in memory associated with aging.

As described above, the food product of the present invention may be provided in a mixed form of the hop oxidation-reaction product and a food ingested as a daily ingested food or dietary supplement, while the hop oxidation-reaction product may be contained in health foods or functional foods, preferably foods containing one, two, or more other components intended for the maintenance, enhancement, and/or improvement of a cognitive function. Alternatively, the food product of the present invention containing the hop oxidation-reaction product may be further supplemented with one, two, or more other components intended for the maintenance, enhancement, and/or improvement of a cognitive function. Examples of the other component intended for the maintenance, enhancement, and/or improvement of a cognitive function are as described above.

The form of the "food product" is not particularly limited, but it may be, for example, in the form of a beverage or in a semi-liquid or gelatinous form. Moreover, examples of the form of the dietary supplement include tablets and capsules which are respectively produced by compressing the hop oxidation-reaction product in a dry powder form mixed and kneaded with, for example, excipients and binders into tablets and by enclosing the hop oxidation-reaction product in a dry powder form mixed and kneaded with, for example, excipients and binders in capsules.

Examples of the food product provided according to the present invention include, but are not limited to, carbohydrate-containing foods and drinks such as cooked rice, noodles, breads, and pasta; various types of confections including Western confections such as cookies and cakes, Japanese confections such as manju buns and yokan jelly, candies, gums, and cooled or frozen sweets such as yoghurt and custard pudding; alcoholic beverages such as whisky, bourbon whisky, spirits, liqueur, wine, fruit wine, Japanese sake, Chinese liquor, Japanese shochu, beer, non-alcoholic beer containing not more than 1% alcohol by volume, sparkling liquor, other miscellaneous liquors, and white liquor highball; non-alcoholic beverages such as fruit juice beverage, vegetable juice beverage, fruit and vegetable juice beverage, soft drink, carbonated drink, milk, soybean milk, milk beverage, drinkable yoghurt, drinkable jelly, coffee, hot chocolate, tea drink, nutritional beverage, sports drink, mineral water, flavored water, and non-alcoholic beer-taste beverage; and processed foods (including delicacies) using eggs, fishes, or animal meats (including giblets and entrails such as liver). Mineral water includes both carbonated and non-carbonated water.

Tea drink includes all of fermented tea, semi-fermented tea and non-fermented tea and examples of tea drink include black tea, green tea, barley tea, green tea with roasted brown rice, green leaf tea, Gyokuro green tea, roasted green tea, Oolong tea, turmeric herbal tea, Pu'er tea, rooibos tea, rose tea, chrysanthemum tea, ginkgo leaf tea, and herbal tea (for example, mint tea and jasmine tea).

Examples of fruits that are used in fruit juice beverages and fruit and vegetable juice beverages include apple, orange, grape, banana, pear, peach, mango fruit, acai berry, blue berry, and plum. Moreover, examples of vegetables that are used in vegetable juice beverages and fruit and vegetable juice beverages include tomato, carrot, celery, pumpkin, cucumber, and water melon.

The hop oxidation-reaction product, which is an active ingredient of the present invention, uses a hop-derived component which has been long ingested as a food by human, and thus the hop oxidation-reaction product has a low toxicity, which enables the use of the hop oxidation-reaction product in a safe manner on mammals in need thereof (for example, human, mouse, rat, rabbit, dog, cat, cow, horse, pig, and monkey). The amount of the hop oxidation-reaction product to be ingested or administered can be determined depending on, for example, the gender, age and body weight of a recipient, conditions, administration time, dosage form, route of administration, and other agents to be combined. In the present invention, an exemplary amount (on the dry-mass basis) of the hop oxidation-reaction product provided to an adult (with a body weight of 50 kg) in a single ingestion or administration includes an amount of 8 to 1700 mg (preferably 160 to 1000 mg), while an exemplary amount (on the dry-mass basis) of the S-fraction provided to an adult (with a body weight of 50 kg) in a single ingestion or administration includes an amount of 1 to 200 mg (preferably 20 to 120 mg).

For the maintenance, enhancement, and improvement of a cognitive function in a healthy person, an exemplary amount (on the dry-mass basis) of the hop oxidation-reaction product provided to an adult (with a body weight of 50 kg) in a single ingestion or administration includes, for example, an amount of 160 to 1000 mg, while an exemplary amount (on the dry-mass basis) of the S-fraction provided to an adult (with a body weight of 50 kg) in a single ingestion or administration includes, for example, an amount of 20 to 120 mg. For the maintenance, enhancement, and improvement of a cognitive function in a person with any reduced cognitive function (including a patient with dementia, a person with suspected mild cognitive impairment, and a person aware of its own mild cognitive impairment), an exemplary amount (on the dry-mass basis) of the hop oxidation-reaction product provided to an adult (with a body weight of 50 kg) in a single ingestion or administration includes, for example, an amount of 8 to 1700 mg, while an exemplary amount (on the dry-mass basis) of the S-fraction provided to an adult (with a body weight of 50 kg) in a single ingestion or administration includes, for example, an amount of 1 to 200 mg.

The hop oxidation-reaction product may be divided into several doses for ingestion or administration according to the conditions of a subject. The hop oxidation-reaction product in the above-described amount can be fed or administered once a week or more frequently (preferably once every three days, more preferably daily) for one month (preferably 3

months, more preferably 6 months) to expect medium- and long-term effects.

EXAMPLES

The present invention will be specifically described by way of examples below, but the present invention is not limited to these examples.

Reference Example 1: Preparation of a hop pellet oxidation-reaction product

A hop pellet oxidation-reaction product was made from Hallertau Perle hops (HPE variety) in the form of a pellet. The hops were ground in a mill and a heating reaction at 80°C was maintained for 24 hours. The obtained product was subjected to the following pre-analysis treatment and then to HPLC analysis.

[Pre-analysis treatment of a product]

Ethanol was added to the collected product to a concentration of 10% (w/v) and extracted at 50°C for one hour. The obtained liquid extract was diluted 10 times with ethanol.

[Components of HPLC apparatus]

Pump: LC-10ADvp x3 (SHIMADZU)
Degasser: DGU-20A5 (SHIMADZU)
System controller: CBM-20A (SHIMADZU)
Autosampler: SIL-20ACHT (SHIMADZU)
Column oven: CTO-20AC (SHIMADZU)
Photodiode array detector: SPD-M20A (SHIMADZU)
Waveform analysis software: LCSolution (SHIMADZU)

[HPLC conditions]

Column: Alltima C18 2.1mm I.D. x 100 mm, particle size: 3 μm
Flow rate: 0.6 mL/min
Elution solvent A: water/phosphoric acid, 1000/0.2, (v/v) + EDTA (free), 0.02% (w/v)
Elution solvent B: acetonitrile
Elution solvent C: water
Injection volume: 3 μL
Column temperature: 40°C
Detection wavelength: 270 nm (oxidation products: iso-α-acids, α-acids, β-acids)
Gradient program:

[Table 1]

| | Composition of mobile phase (%) | | |
|---|---|---|---|
| Time (min) | A | B | C |
| 0 | 90 | 10 | 0 |
| 26.67 | 48 | 52 | 0 |
| 30 | 25 | 75 | 0 |
| 32.67 | 15 | 85 | 0 |
| 37.67 | 15 | 85 | 0 |
| 37.68 | 0 | 10 | 90 |

(continued)

| | Composition of mobile phase (%) | | |
|---|---|---|---|
| Time (min) | A | B | C |
| 41.3 | 0 | 10 | 90 |
| 41.31 | 90 | 10 | 0 |
| 51 | stop | | |
| Washing and equilibration steps started after 37.68 min. | | | |

The above-described products was subjected to a measurement under the above-described analytical conditions to analyze the chromatogram-waveform at detected wavelength of 270 nm, and the ratios (%) of the area values of peaks corresponding to α-acids, β-acids, and iso-α-acids to the sum of the area values of all peaks (mAU-min) were calculated. Areas corresponding to solvent peaks and a negative peak caused by injection shock were excluded from the subject areas of the waveleform analysis. A HPLC chromatogram of the analysis of the above-described products was as shown in FIG. 1.

Example 1: Preparation of a hop oxidation-reaction product

(1) Hop oxidation step

Hallertau Perle hops (HPE variety) were ground in a mill and the obtained ground hops were heated with stirring at 60°C for about 120 hours under atmospheric pressure. Water was added to the obtained heated hops (aged hop pellets) to give a solid concentration of 5% (w/v) and the resulting mixture was subjected to extraction at 50°C for 30 minutes. The obtained liquid extract was separated into solid and liquid phases by decantation to obtain a solids-free liquid (with a degree Brix of about 2).

(2) Activated charcoal treatment step

To the solids-free liquid obtained in the above-described procedure (1), activated charcoal (Y180C, manufactured by Ajinomoto Fine-chemical Co., Inc.; at 0.5% (w/v) relative to the solids-free liquid) and polyvinylpolypyrrolidone (Polyclar 10, manufactured by ISP Japan Ltd.; at 0.4% (w/v) relative to the solids-free liquid) were added and the resulting mixture was left to stand for 2 hours. The obtained liquid mixture was supplemented with a filter aid (diatom earth) and then filtrated to obtain a filtrate (with a degree Brix of about 1.5). The obtained filtrate was used as an aqueous extract of a hop oxidation-reaction product in the following examples.

(3) Analysis of ingredients of a hop oxidation-reaction product

For the filtrate (the aqueous extract of the hop oxidation-reaction product) obtained in the above-described procedure (2), HPLC-MSMS analysis was performed under the following conditions to measure the contents of various ingredients contained in the hop oxidation-reaction product. It is known that scorpio-humulinol A, scorpio-cohumulinol A, tricyclooxyisohumulone A, and tricyclooxyisocohumulone A are contained in a hop oxidation-reaction product as oxidation products of α-acids, iso-α-acids, or β-acids (Biosci. Biotechnol. Biochem., 2015 (79): 1684-1694; J. Agric. Food Chem., 2015, 63: 10181-10191). Moreover, a standard used in the analysis was prepared in accordance with the method described in J. Agric. Food Chem., 2015, 63: 10181-10191 and J. Nat. Prod., 2014, 77, 1252-1261.

[HPLC conditions]

Column: Unison UK-C18 100 x 2mm i.d., particle size: 3μm
Flow rate: 0.25 mL/min
Column temperature: 40°C
Mobile phase A: 1% formic acid in water
Mobile phase B: 1% formic acid in acetonitrile
Injection volume: 3 μL
Gradient:

From 0 to 30 min, 15 to 31% B
From 30 to 40 min, 31 to 80% B
From 40 to 43 min, 80% B
Followed by washing and equilibration steps

[MSMS conditions]

Mass spectrometer: AB SCIEX 4000Q Trap
Ion source: ESI in negative mode
Ion spray voltage: -4500 V
Analytical parameters:

[Table 2]

| Table 2: Analytical parameters for each compound | | | | | |
|---|---|---|---|---|---|
| Compound | Q1 mass (amu) | Q3 mass (amu) | Declustering potential (V) | Collision energy (V) | Cell exit potential (V) |
| Scorpio-humulinol A | 392.91 | 194.90 | -100 | -38 | -13 |
| Scorpio-cohumulinol A | 378.91 | 180.90 | -100 | -38 | -13 |
| Tricyclooxyisohumulone A | 376.90 | 124.80 | -115 | -50 | -19 |
| Tricyclooxyisocohumulone A | 362.90 | 110.80 | -60 | -50 | -17 |

The above-described procedures (1) and (2) were repeated three times to obtain filtrates and the contents of scorpio-humulinol A, scorpio-cohumulinol A, tricyclooxyisohumulone A, and tricyclooxyisocohumulone A in the filtrates were measured. As a result, the ratios of the total amount of tricyclooxyisohumulone A and tricyclooxyisocohumulone A to the total amount of scorpio-humulinol A and scorpio-cohumulinol A ([tricyclooxyisohumulone A + tricyclooxyisocohumulone A] / [scorpio-humulinol A + scorpio-cohumulinol A]) were as described below:

Lot 1, 3.2;
Lot 2, 6.8;
Lot 3, 12.1.

Consequently, it was found that the ratio of the total amount of tricyclooxyisohumulone and tricyclooxyisocohumulone to the total amount of scorpio-humulinol and scorpio-cohumulinol was from about 2 to 20 in the hop oxidation-reaction product.

<u>Example 2: Evaluation of the effects of ingestion of the hop oxidation-reaction product on cognitive functions (1)</u>

(1) Preparation of placebo and test foods

Hard capsules (Size number 1) each filled with 257 mg of crystalline cellulose alone were used as placebo. Hard capsules each filled with 100 mg of crystalline cellulose and 153 mg of a dried form of the aqueous extract of the hop oxidation-reaction product obtained in the procedure (2) of Example 1 were used as a test food. The hard capsules used for the respective foods had the same color and shape so that the foods were indistinguishable in appearance from each other. A single capsule of the test food contains 17.5 mg of the S-Fr on the dry-mass basis.

(2) Test conditions

Ten healthy non-smoking men and women not younger than 40 years old were recruited and were randomly assigned to groups of five subjects, the placebo group and the test food group. Two capsules of the placebo were used daily for the subjects in the placebo group, and two capsules of the test food were used daily for the subjects in the test food group (two capsules of the test food contain 35 mg of the S-Fr on the dry-mass basis). The test schedule is as depicted in

FIG. 2. Specifically, the test subjects were not fed with the respective foods but received cognitive function assessments on study day 1. On study day 3, the test subjects were fed with the respective foods and one hour later received cognitive function assessments. On study day 4, the test subjects were only fed with the respective foods. On study day 5, the test subjects were fed with the respective foods and one hour later received cognitive function assessments. Additionally, the test subjects were not allowed to ingest any hopcontaining food or drink during the test period and also not allowed to ingest any caffeine-containing food or drink before the cognitive function assessments on study days 1, 3, and 5.

(3) Methods for evaluation of cognitive functions

The Trail Making Test Part A (hereinafter referred to as TMT), the Letter-Number Sequencing Test (hereinafter referred to as LNS), and CANTAB from Cambridge Cognition Holdings Plc were used as the cognitive function assessments. CANTAB is software with which multiple cognitive function assessments can be implemented. Among those assessments, the Pattern Recognition Memory (hereinafter referred to as PRM), the Spatial Working Memory (hereinafter referred to as SWM), and the Paired Associates Learning (hereinafter referred to as PAL) were used. The order of those assessments was as follows: TMT, PRM, SWM, PAL, and LNS. The test subjects underwent those cognitive function assessments during the morning, which were started after the test subjects entered an examination room and then rested for 10 minutes. Details of the respective assessment methods are as described below. Each of the assessment methods is a widely accepted method for the evaluation of cognitive functions.

(i) TMT

TMT is a method of assessing visual searching and processing speed and sustainability of attention. Specifically, a sheet of paper on which numbers 1-25 were printed was used and the test subjects drew a line in numerical order starting at number 1 and ending on number 25 and the time required to complete the task was measured. A shorter period to complete the task means a faster processing speed, indicating sustained attention and concentration.

(ii) PRM

PRM is a method of assessing visual pattern recognition memory performance. Specifically, the test subjects first memorized 10 different patterns displayed on the screen of a tablet PC in a random order. After all of the patterns were presented, two different patterns (one of the previously presented patterns and a novel pattern) were displayed in the middle of the screen and the test subjects were required to touch the previously presented pattern. Touching the previously presented pattern is regarded as a correct answer and a more number of correct answers mean higher visual pattern recognition memory performance, that is, higher visual memory performance.

(iii) SWM

SWM is a method of assessing spatial working memory performance. Specifically, the test subjects were required to sequentially touch and open 12 boxes displayed on the screen of a tablet PC. Only one of the 12 boxes contained inside a blue square token, which the test subjects were required to find in one test cycle and the test cycle was repeated 12 times. Once a box containing a blue square token is discovered, the same box will never contain a blue square token in the subsequent test cycles. Touching the same box twice or more in one test cycle increased the count of "within errors" and touching the box which had already contained a blue square token in the 12 test cycles increased the count of "between errors". A fewer number of "within errors" and "between errors" mean higher spatial working memory performance.

(iv) PAL

PAL is a method of assessing visuospatial recognition-associated memory performance and learning ability. Specifically, 12 boxes each hiding one pattern were displayed on the screen of a tablet PC. The boxes were opened one by one in a random order to present patterns and the test subjects were required to memorize the patterns and the locations of the boxes containing the patterns. After all of the boxes were opened, the patterns were displayed in the middle of the screen, one at a time and the test subjects were required to touch the box in which the pattern was originally contained. The test was continued until the locations of the boxes containing the 12 patterns were correctly answered and the number of incorrect answers was counted as the number of errors. A fewer number of errors means higher spatial recognition-associated memory performance and higher learning ability.

(v) LNS

LNS is a method of assessing linguistic working memory performance. A test examiner read a sequence of randomly mixed numbers and alphabet letters and the test subjects were required to memorize the sequence, reorder the numbers and letters in the sequence, and then pronounce the numbers in ascending order and then the letters in alphabetical order. Pronouncing the numbers and letters in correct orders is regarded as a correct answer and a more number of correct answers mean higher linguistic working memory performance. An example of the used questionnaires is shown in FIG. 3 (sequences read by a test examiner are presented on left lines in the questionnaire, while the answers to the questions are presented on right lines).

(4) Results

The background information of the test subjects in both groups is presented in Table 3. It was confirmed that there were no deviations in age and gender between the groups.

[Table 3]

| Table 3: Backgrounds of test subjects | | |
|---|---|---|
| | Placebo group | Test food group |
| Age (Mean ± SD) | 47.8 ± 6.0 | 45.2±3.4 |
| Gender (number of males/number of females) | 3/2 | 2/3 |

For the results of TMT, the result of the first trial was taken as reference values and the results of the second and third trials are indicated as variation values changed from the result of the first trial in FIG. 4. According to FIG. 4, it was confirmed that the time required to complete the task was shorter in both the second and the third trials in the test food group, as compared to that in the placebo group. This indicated that the ingestion of the hop oxidation-reaction product enhanced attention and concentration.

For the results of PRM, the result of the first trial was taken as reference values and the results of the second and third trials are indicated as variation values changed from the result of the first trial in FIG. 5. According to the results shown in FIG. 5, it was confirmed that the number of correct answers was increased in both the second and the third trials in the test food group, as compared to that in the placebo group. This indicated that the ingestion of the hop oxidation-reaction product enhanced visual memory performance.

For the results of SWM, the result of the first trial was taken as reference values and the results of the second and third trials are indicated as variation values changed from the result of the first trial in FIGS. 6 and 7. According to the results shown in FIGS. 6 and 7, it was confirmed that the number of errors, including the within errors (FIG. 6) and the between errors (FIG. 7), was decreased in both the second and the third trials in the test food group, as compared to that in the placebo group. This indicated that the ingestion of the hop oxidation-reaction product enhanced spatial working memory performance.

For the results of PAL, the result of the first trial was taken as reference values and the results of the second and third trials are indicated as variation values changed from the result of the first trial in FIG. 8. According to the results shown in FIG. 8, it was confirmed that the number of errors was decreased in both the second and the third trials in the test food group, as compared to that in the placebo group. These indicated that the ingestion of the hop oxidation-reaction product enhanced spatial recognition-associated memory performance and learning ability.

For the results of LNS, the result of the first trial was taken as reference values and the results of the second and third trials are indicated as variation values changed from the result of the first trial in FIG. 9. According to the results shown in FIG. 9, it was confirmed that the number of correct answers was increased in both the second and the third trials in the test food group, as compared to that in the placebo group. This indicated that the ingestion of the hop oxidation-reaction product enhanced linguistic working memory performance.

In all the evaluation items, enhancement by the ingestion of the hop oxidation-reaction product was observed. These results showed that ingestion of a hop oxidation-reaction product can be expected to enhance a wide range of cognitive functions.

Example 3: Evaluation of the effects of ingestion of the hop oxidation-reaction product on cognitive functions (2)

The effects of a hop oxidation-reaction product on short-term memory and spatial memory were evaluated in this example.

(1) Method for evaluation of cognitive functions (Y-maze test)

When a mouse is placed in a certain space, a mouse naturally has a predilection for exploring novelty and thus has a tendency to select a route different from the most recently selected route in cases where the mouse remembers the most recently selected route. Thus, in cases where a mouse is placed in a Y-shaped maze with three arms of equal width and equal length, the mouse usually enters an arm different from the arm that the mouse most recently entered. The Y-maze test is a test that utilizes the above-described tendency of mice and is used for the evaluation of short-term memory performance and spatial memory performance, which are indices of cognitive functions.

In this test, a Y-shaped maze with three arms of 25 cm arm length, 20 cm wall height, and 5 cm floor width, attached at 120 degree angles, was used as a Y-maze test apparatus.

Each mouse was placed on the tip of any of the arms in the Y-shaped maze and was allowed to freely explore the maze for 8 minutes, during which the sequence of arm entries by the mouse was recorded. The selection and entering into different arms by a mouse in three consecutive times is referred to as spontaneous alternation behavior. The total number of entering into arms and the spontaneous alternation number in a particular time period were counted and the spontaneous alternation rate (%) was calculated based on the following formula (1).

[Math 1]

$$\text{Spontaneous alternation rate (\%)} = \frac{\text{(Spontaneous alternation number)}}{\text{(Total entry number} - 2)} \times 100 \qquad (1)$$

A higher spontaneous alternation rate means better maintained short-term memory.

(2) Effect of ingestion of a hop oxidation-reaction product

The hop oxidation-reaction product was administered as the S-Fr by gavage to 6-week-old male CD-1 mice (obtained from Japan SLC, Inc.) at a dose of 0 mg (a dilution solvent), 1 mg, 3 mg, or 10 mg (on the dry-mass basis) per kg of body weight. The administered hop oxidation-reaction product containing the S-Fr was prepared immediately prior to use by adding distilled water to a dried form of the extract of the hop oxidation-reaction product obtained in the procedure (2) of Example 1. Forty minutes after the administration, scopolamine hydrochloride (manufactured by Sigma-Aldrich Co. LLC) was administered intraperitoneally at a dose of 0.85 mg/kg body weight to induce memory impairment and produced amnesia mouse models. Twenty minutes after the intraperitoneal administration of scopolamine (SCP), the Y-maze test was carried out (FIG. 10). The experiment was performed on 10 mice in each group to obtain the average values and the standard errors.

FIG. 11 and FIG. 12 show the total entry numbers and the spontaneous alternation rates, respectively. According to the results shown in FIGS. 11 and 12, it was confirmed that the administration of the hop oxidation-reaction product had no effect on total entry numbers, that is, no effect on locomotor activity (FIG. 11), but that the hop oxidation-reaction product increased the spontaneous alternation rate, namely to promote maintenance of short-term memory, in the hop oxidation-reaction product-administered groups, as compared with the control-administrated group (FIG. 12). These indicated that the hop oxidation-reaction product containing the S-Fr successfully improved cognitive functions.

Example 4: Evaluation of the effects of ingestion of the hop oxidation-reaction product on cognitive functions (3)

The effects of a hop oxidation-reaction product on long-term memory and episodic memory were evaluated in this example.

(1) Method for evaluation of cognitive functions (novel object recognition test)

When a mouse finds a novel object, the mouse naturally shows exploratory behaviors toward the novel object, which include, for example, approaching the object, identifying the shape of the object, and sniffing the object. In this case, another object which the mouse remembers is not explored or is explored only for a shorter time period, as compared to the novel object. The novel object recognition test utilizes this tendency of mice.

Two wooden blocks X and Y of the same shape as large as golf balls were placed in a container (38.5 cm x 38.5 cm floor, 40 cm height) so that the wooden blocks are each 4 cm apart from neighboring corners in the direction toward the center of the floor. Each mouse was placed in the container and was allowed to freely explore the container for 10 minutes, after which the mouse was returned to a home cage. This step is referred to as acquisition trial.

After 24 hours, the mouse was again placed in the same container, except that the wooden block Y that had been presented 24 hours earlier was replaced with a golf ball Z. The mouse was allowed to freely explore the container for 5 minutes, during which time spent touching those two objects was separately measured. This step is referred to as test trial.

Maintenance of memory is evaluated based on the difference in length of time spent for exploratory behaviors toward the objects X and Z.

A short time interval between an acquisition trial and a test trial causes a mouse to show exploratory behaviors toward a novel object (in this study, the golf ball Z) for a longer time period, while the preference for a novel object decreases in association with increase in the time interval between an acquisition trial and a test trial. Therefore, it is commonly understood that the change in behavior toward novelty reflects "the memory regarding the shapes of objects existing in an acquisition trial".

Time spent exploring the object existing in the acquisition trial and time spent exploring the novel object existing in the test trial were measured and the discrimination index (DI) was calculated based on the following formula (2).

[Math 2]

$$\mathrm{DI} = \frac{(\text{Time spent exploring a novel object (sec.)} - \text{time spent exploring an object existing in an acquisition trial (sec.)})}{(\text{Total exploration time (sec.)})} \quad (2)$$

A higher discrimination index means better maintained memory of the objects existing in an acquisition trial, suggesting better maintained long-term memory. The scheme of the experiment was as shown in FIG. 13.

(2) Effect of ingestion of a hop oxidation-reaction product

The hop oxidation-reaction product was administered as the S-Fr by gavage to 6-week-old male CD-1 mice in groups of 10 at a dose of 0 mg (a dilution solvent), 1 mg, or 3 mg (on the dry-mass basis) per kg body weight. The above-described hop oxidation-reaction product was prepared by the same procedure as the procedure (2) of Example 3. Sixty minutes after the administration, the mice underwent an acquisition trial. After 24 hours, the hop oxidation-reaction product was administered by gavage to each of the individuals at the same dose as used in the acquisition trial, and 60 minutes later the mice underwent a test trial. The result was as shown in FIG. 14.

The groups of mice intragastrically administered with the hop oxidation-reaction product showed high discrimination index values and the increase in discrimination index seemingly tended to be dose-dependent. The administration of the hop oxidation-reaction product containing the S-Fr was indicated to contribute to the maintenance of memory regarding the shapes of the objects in the acquisition trial, that is, to the enhancement of long-term memory performance.

Time spent exploring the novel object and time spent exploring the familiar object were measured and the ratios of time spent for the exploration (exploration time ratios (%)) were calculated based on the following formulae (3) and (4) (FIG. 15).

[Math 3]

$$\text{Ratio of time spent exploring a familiar object} = \frac{(\text{Time spent exploring an object X})}{(\text{Total exploration time})} \times 100 \quad (3)$$

[Math 4]

$$\text{Ratio of time spent exploring a novel object} = \frac{(\text{Time spent exploring an object Z})}{(\text{Total exploration time})} \times 100 \quad (4)$$

Seemingly, the ratio of time spent exploring the novel object tended to be increased in a dosage-dependent manner by administering the hop oxidation-reaction product containing the S-Fr.

<u>Example 5: Evaluation of the effects of ingestion of the hop oxidation-reaction product on cognitive functions (4)</u>

Alzheimer's disease models were assessed for short-term memory and spatial memory in this example.

(1) Preparation of Alzheimer's disease model

The oligomer hypothesis for Alzheimer's disease asserts that amyloid β, one of the causative substances of Alzheimer's disease, forms neurotoxic soluble oligomeric assemblies in the brain. ADDLs (Aβ-derived diffusible ligands), a kind of oligomeric assemblies of amyloid β, were identified as ligands that impaired synaptic plasticity and intraventricular ADDL administration exhibits an effect to impair cognitive functions. Therefore, mice administered with an ADDL can be used as models of cognitive decline and diseases that are developed by accumulation of waste metabolites in the brain, particularly Alzheimer-type dementia (Nobuhisa Iwata, and Takaomi Saido (2010) "Unraveling the Mystery of Alzheimer's disease", Chugai-Igakusha, pp. 173-180).

Amyloid β-peptide (1-42) (manufactured by Peptide Institute, Inc.) was dissolved in hexafluoroisopropanol (HFIP, manufactured by Wako Pure Chemical Industries, Ltd.) to a concentration of 1 mM. The solution was left to stand at room temperature for 30 minutes and then the HFIP was removed from the solution by evaporation. The remaining peptide was dissolved in dimethyl sulfoxide (DMSO, manufactured by Wako Pure Chemical Industries, Ltd.) to a concentration of 5 mM. The solution was diluted in phosphate-buffered saline (PBS) to a peptide concentration of 100 μM and the resulting dilution was left to stand at 4°C for 24 hours to polymerize the amyloid β. The solution was centrifuged at 10,000 rpm for 15 minutes to obtain a supernatant as an ADDL solution.

Five-week-old male CD-1 mice (obtained from Charles River Laboratories Japan, Inc.) in groups of 12 were anesthetized with Somnopentyl (manufactured by Kyoritsuseiyaku Corporation) and administered with 5 μL of the above-described ADDL solution per each lateral ventricle to prepare Alzheimer's disease mouse models. Moreover, the same procedure was repeated to prepare sham-operated mice administered with PBS into their both lateral ventricles.

(2) Effect of ingestion of a hop oxidation-reaction product

(i) Y-maze test

The hop oxidation-reaction product was administered as the S-Fr by oral gavage to Alzheimer's disease mouse models at a dose of 0 mg (a dilution solvent), 1 mg, or 10 mg (on the dry-mass basis) per kg body weight. The above-described hop oxidation-reaction product was prepared by the same procedure as the procedure (2) of Example 3. The mice were assessed for cognitive functions 60 minutes after the single administration by the same Y-maze test as the procedure (1) of Example 3.

FIG. 16 and FIG. 17 show the total entry numbers and the spontaneous alternation rates, respectively. According to the result shown in FIG. 16, it was confirmed that the administration of the hop oxidation-reaction product had no effect on change in total entry numbers, that is, no effect on locomotor activity (FIG. 16), but that the hop oxidation-reaction product increased the spontaneous alternation rate, namely to promote cognitive functions such as maintenance of short-term memory, in the hop oxidation-reaction product-administered groups, as compared with the controladministred group. Moreover, the effect seemingly tended to depend on the dosage of the S-Fr (FIG. 17).

(ii) Novel object recognition test

The hop oxidation-reaction product was administered as the S-Fr by oral gavage to Alzheimer's disease mouse models at a dose of 0 mg (a dilution solvent) or 10 mg (on the dry-mass basis) per kg body weight. The above-described hop oxidation-reaction product was prepared by the same procedure as the procedure (2) of Example 3. Sixty minutes after the administration, the mice underwent an acquisition trial of the same novel object recognition test as described in the procedure (1) of Example 4. After 24 hours, the hop oxidation-reaction product was administered by gavage to each of the individuals at the same dose as used in the acquisition trial, and 60 minutes later the mice underwent a test trial. The result was as shown in FIG. 18.

The groups of mice intragastrically administered with the hop oxidation-reaction product showed high discrimination index values. The administration of the hop oxidation-reaction product containing the S-Fr was indicated to contribute to the maintenance of memory regarding the shapes of the objects in the acquisition trial, that is, to the enhancement of long-term memory performance.

The ratios of time spent for the exploration (exploration time ratios (%)) were calculated similarly to Example 4 (FIG. 19). Seemingly, the ratio of time spent exploring the novel object tended to be increased by administering the hop oxidation-reaction product containing the S-Fr.

The results of the tests (i) and (ii) indicated that the ingestion of the hop oxidation-reaction product containing the S-Fr even in a short time period ameliorated conditions associated with accumulation of waste metabolites in the brain, such as cognitive decline, reduction in establishment, maintenance, and recovery of memory, and, furthermore, dementia including Alzheimer's disease.

## Claims

1. A composition for use in a method of maintaining, enhancing, and/or improving a cognitive function, comprising a hop oxidation-reaction product, wherein oxidation is performed by heating hop in the air at a temperature between 60 °C and 100 °C, wherein said hop contains lupulin glands, and wherein said hop oxidation-reaction product is provided as an extract in water, wherein the extraction temperature is 60 °C or lower, wherein the hop oxidation-reaction product has a ratio of the total amount of tricyclooxyisohumulone A and tricyclooxyisocohumulone A to the total amount of scorpio-humulinol A and scorpio-cohumulinol A (on the dry-mass basis) from 2 to 20.

2. The composition for use according to claim 1, wherein the cognitive function is memory function.

3. The composition for use according to claim 1, wherein the cognitive function is attention or concentration function.

4. The composition for use according to any one of claims 1 to 3, which is a food composition.

5. The composition for use according to any one of claims 1 to 4, which is in a single unit package suitable for a single ingestion.

6. The composition for use according to any one of claims 1-5, which comprises the hop oxidation-reaction product in an amount of 8 to 1700 mg on the dry-mass basis for a single ingestion.

7. The composition for use according to any one of claims 1 to 6, for use in the treatment, prevention, or amelioration of memory impairment, disorientation, and/or cognitive impairment.

8. A hop oxidation-reaction product or a food product or an agent for use in maintaining, enhancing, and/or improving a cognitive function, wherein oxidation is performed by heating hop in the air at a temperature between 60 °C and 100 °C, wherein said hop contains lupulin glands, and wherein said hop oxidation-reaction product is provided as an extract in water, wherein the extraction temperature is 60 °C or lower, wherein the hop oxidation-reaction product has a ratio of the total amount of tricyclooxyisohumulone A and tricyclooxyisocohumulone A to the total amount of scorpio-humulinol A and scorpio-cohumulinol A (on the dry-mass basis) from 2 to 20.

## Patentansprüche

1. Zusammensetzung zur Verwendung in einem Verfahren zur Aufrechterhaltung, Steigerung und/oder Verbesserung einer kognitiven Funktion, umfassend ein Hopfen-Oxidationsreaktionsprodukt, wobei Oxidation durch Erhitzen von Hopfen bei einer Temperatur zwischen 60 °C und 100 °C an der Luft durchgeführt wird, wobei der Hopfen Lupulindrüsen enthält und wobei das Hopfen-Oxidationsreaktionsprodukt als ein Extrakt in Wasser bereitgestellt wird, wobei die Extraktionstemperatur 60°C oder weniger beträgt, wobei das Hopfen-Oxidationsreaktionsprodukt ein Verhältnis von der Gesamtmenge an Tricyclooxy-Isohumulon A und Tricyclooxy-Iso-Cohumulon A zur Gesamtmenge an Skorpio-Humulinol A ("scorpio-humulinol A") und Skorpio-Cohumulinol A ("scorpio-cohumulinol A") (auf Basis des Trockengewichts) von 2 bis 20 aufweist.

2. Zusammensetzung zur Verwendung nach Anspruch 1, wobei die kognitive Funktion die Gedächtnisfunktion ist.

3. Zusammensetzung zur Verwendung nach Anspruch 1, wobei die kognitive Funktion die Aufmerksamkeit oder Konzentrationsfunktion ist.

4. Zusammensetzung zur Verwendung nach irgendeinem der Ansprüche 1 bis 3, die eine Lebensmittelzusammensetzung ist.

5. Zusammensetzung zur Verwendung nach irgendeinem der Ansprüche 1 bis 4, die in einer Einzelpackung vorliegt, die für eine einmalige Einnahme geeignet ist.

6. Zusammensetzung zur Verwendung nach irgendeinem der Ansprüche 1-5, die das Hopfen-Oxidationsreaktionsprodukt in einer Menge von 8 bis 1700 mg auf Basis des Trockengewichts zur einmaligen Einnahme umfasst.

7. Zusammensetzung zur Verwendung nach irgendeinem der Ansprüche 1 bis 6, zur Verwendung in der Behandlung, Vorbeugung oder Verbesserung von Gedächtnisstörung, Desorientierung und/oder kognitiver Störung.

8. Hopfen-Oxidationsreaktionsprodukt oder ein Lebensmittelprodukt oder ein Agens zur Verwendung in der Aufrechterhaltung, Steigerung und/oder Verbesserung einer kognitiven Funktion, wobei Oxidation durch Erhitzen von Hopfen bei einer Temperatur zwischen 60 °C und 100 °C an der Luft durchgeführt wird, wobei der Hopfen Lupulindrüsen enthält und wobei das Hopfen-Oxidationsreaktionsprodukt als ein Extrakt in Wasser bereitgestellt wird, wobei die Extraktionstemperatur 60°C oder weniger beträgt, wobei das Hopfen-Oxidationsreaktionsprodukt ein Verhältnis von der Gesamtmenge an Tricyclooxy-Isohumulon A und Tricyclooxy-Iso-Cohumulon A zur Gesamtmenge an Skorpio-Humulinol A ("scorpio-humulinol A") und Skorpio-Cohumulinol A ("scorpio-cohumulinol A") (auf Basis des Trockengewichts) von 2 bis 20 aufweist.

**Revendications**

1. Composition destinée à être utilisée dans un procédé de maintien, de renforcement, et/ou d'amélioration d'une fonction cognitive, comprenant un produit de réaction d'oxydation du houblon, dans laquelle l'oxydation est mise en oeuvre en chauffant le houblon dans l'air à une température comprise entre 60 °C et 100 °C, dans laquelle ledit houblon contient des glandes de lupuline, et dans laquelle ledit produit de réaction d'oxydation du houblon est fourni sous la forme d'un extrait dans l'eau, dans laquelle la température d'extraction est de 60 °C ou moins, dans laquelle le produit de réaction d'oxydation du houblon présente un rapport de la quantité totale de tricyclooxyisohumulone A et de tricyclooxyisocohumulone A à la quantité totale de scorpio-humulinol A et de scorpio-cohumulinol A (sur la base de la masse sèche) de 2 à 20.

2. Composition à utiliser selon la revendication 1, dans laquelle la fonction cognitive est la fonction de mémoire.

3. Composition à utiliser selon la revendication 1, dans laquelle la fonction cognitive est la fonction d'attention ou de concentration.

4. Composition à utiliser selon l'une quelconque des revendications 1 à 3, qui est une composition alimentaire.

5. Composition à utiliser selon l'une quelconque des revendications 1 à 4, qui se présente sous la forme d'un conditionnement unitaire adapté à une ingestion unique.

6. Composition à utiliser selon l'une quelconque des revendications 1 à 5, qui comprend le produit de réaction d'oxydation du houblon en une quantité de 8 à 1 700 mg sur la base de la masse sèche pour une ingestion unique.

7. Composition à utiliser selon l'une quelconque des revendications 1 à 6, destinée à être utilisée dans le traitement, la prévention, ou l'amélioration des troubles de la mémoire, de la désorientation, et/ou des troubles cognitifs.

8. Produit de réaction d'oxydation du houblon ou produit alimentaire ou agent destiné à être utilisé pour maintenir, renforcer et/ou améliorer une fonction cognitive, dans lequel l'oxydation est mise en oeuvre en chauffant le houblon dans l'air à une température entre 60 °C et 100 °C, dans lequel ledit houblon contient des glandes de lupuline, et dans lequel ledit produit de réaction d'oxydation du houblon est fourni sous la forme d'un extrait dans l'eau, dans lequel la température d'extraction est de 60 °C ou moins, dans lequel le produit de réaction d'oxydation du houblon présente un rapport de la quantité totale de tricyclooxyisohumulone A et de tricyclooxyisocohumulone A à la quantité totale de scorpio-humulinol A et de scorpio-cohumulinol A (sur la base de la masse sèche) de 2 à 20.

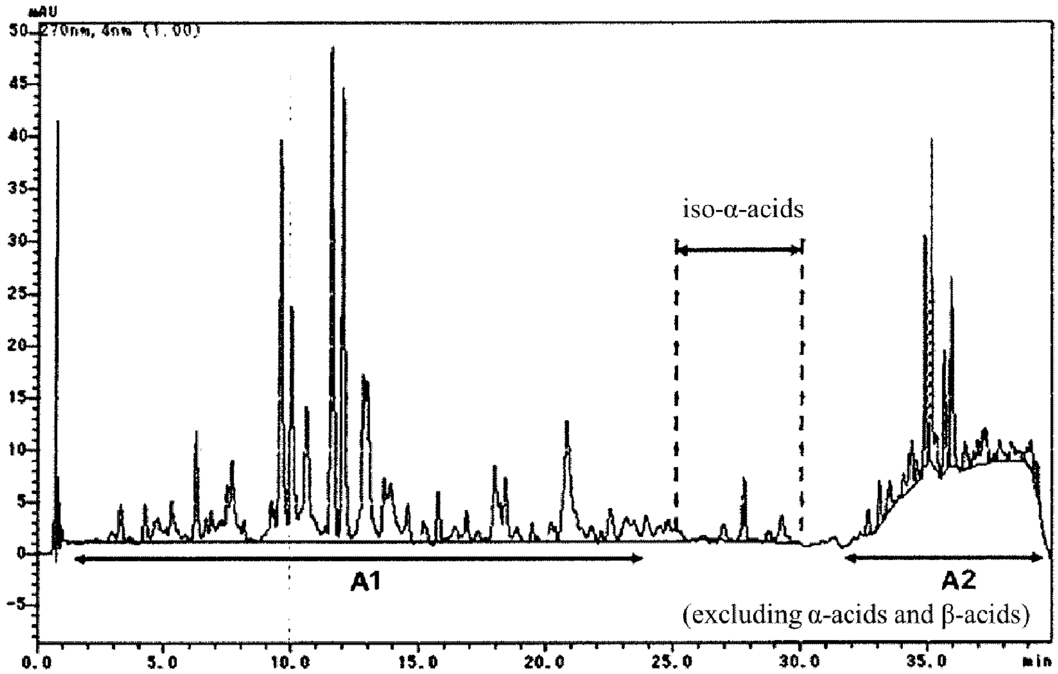


FIGURE 1

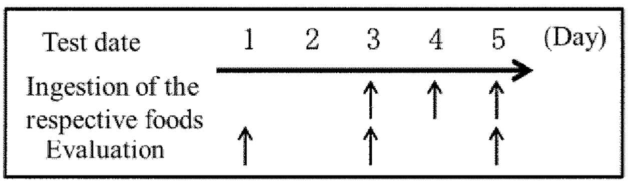


FIGURE 2

| | Trial | Item/Response | | Trial Score (0 or 1) | Item Score (0, 1, 2 or 3) |
|---|---|---|---|---|---|
| 1 | 1 | 5 - L | ( 5 - L ) | | |
| | 2 | G - 3 | ( 3 - G ) | | |
| | 3 | 4 - L | ( 4 - L ) | | |
| 2 | 1 | K - 2 - P | ( 2 - K - P ) | | |
| | 2 | R - B - 7 | ( 7 - B - R ) | | |
| | 3 | 2 - W - A | ( 2 - A - W ) | | |
| 3 | 1 | 3 - M - 5 - O | ( 3 - 5 - M - O ) | | |
| | 2 | V - 4 - 1 - K | ( 1 - 4 - K - V ) | | |
| | 3 | 2 - H - U - 8 | ( 2 - 8 - H - U ) | | |
| 4 | 1 | 7 - J - 1 - X - T | ( 1 - 7 - J - T - X ) | | |
| | 2 | N - 3 - L - F - 5 | ( 3 - 5 - F - L - N ) | | |
| | 3 | L - 1 - J - 4 - W | ( 1 - 4 - J - L - W ) | | |
| 5 | 1 | 3 - U - A - N - 9 - 5 | ( 3 - 5 - 9 - A - N - U ) | | |
| | 2 | 4 - 6 - B - M - 8 - T | ( 4 - 6 - 8 - B - M - T ) | | |
| | 3 | T - 2 - 4 - R - M - 1 | ( 1 - 2 - 4 - M - R - T ) | | |
| 6 | 1 | Y - 2 - E - I - 7 - L - 5 | ( 2 - 5 - 7 - E - I - L - Y ) | | |
| | 2 | H - N - 6 - 3 - Y - 1 - I | ( 1 - 3 - 6 - H - I - N - Y ) | | |
| | 3 | 7 - E - 9 - A - G - 2 - F | ( 2 - 7 - 9 - A - E - F - G ) | | |
| 7 | 1 | N - 8 - U - 4 - F - B - 7 - 1 | ( 1 - 4 - 7 - 8 - B - F - N - U ) | | |
| | 2 | W - 6 - F - 9 - 5 - D - 3 - X | ( 3 - 5 - 6 - 9 - D - F - W - X ) | | |
| | 3 | T - O - 7 - 3 - 2 - K - M - 9 | ( 2 - 3 - 7 - 9 - K - M - O - T ) | | |
| | | | | Total Raw Score (Maximum=21) | |

FIGURE 3

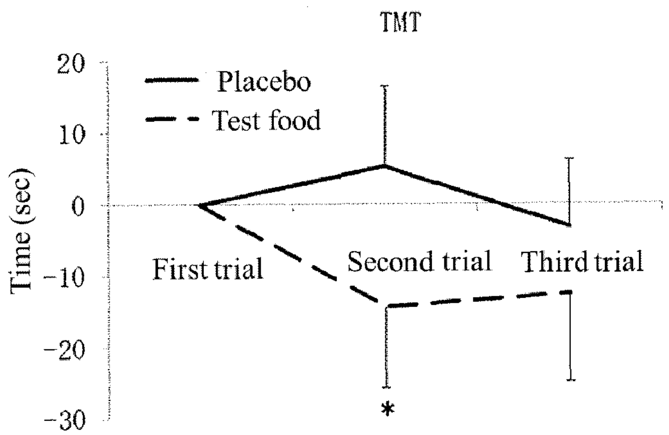


FIGURE 4

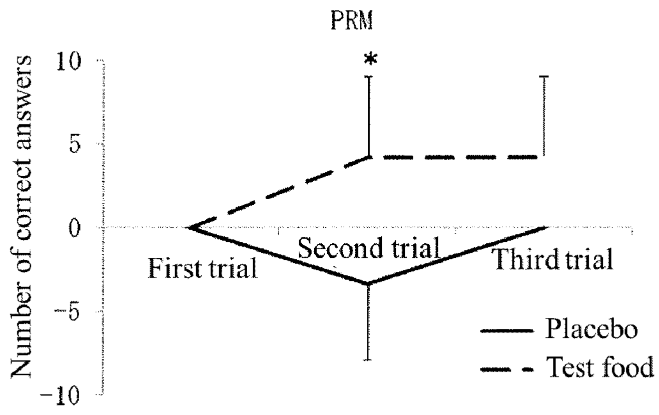


FIGURE 5

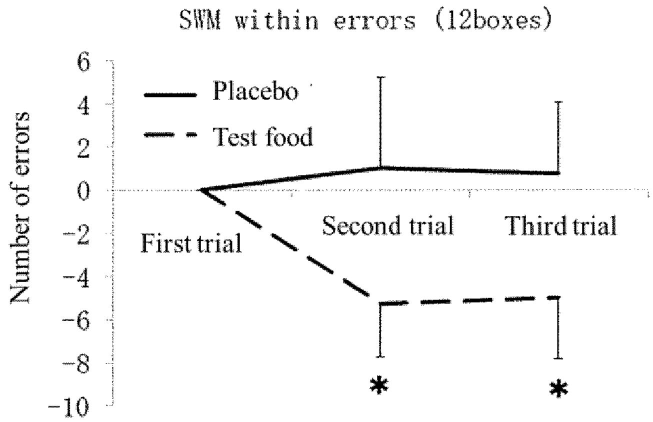


FIGURE 6

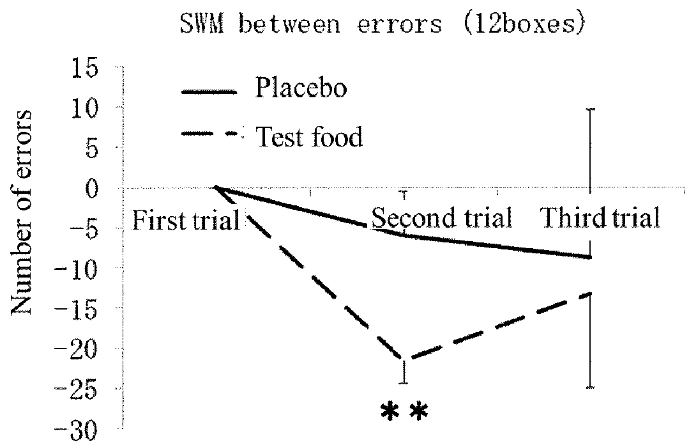


FIGURE 7

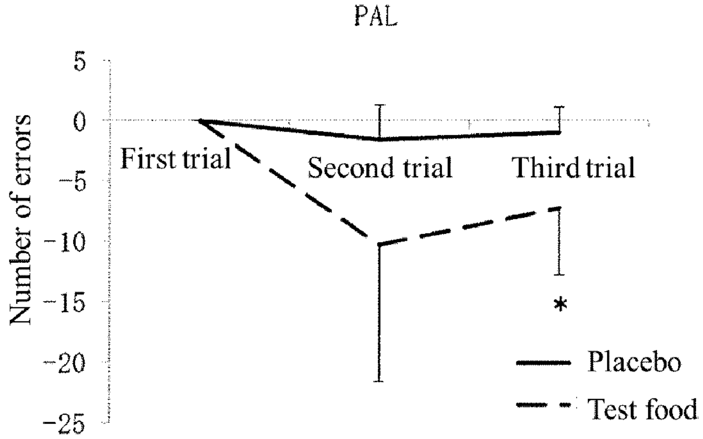


FIGURE 8

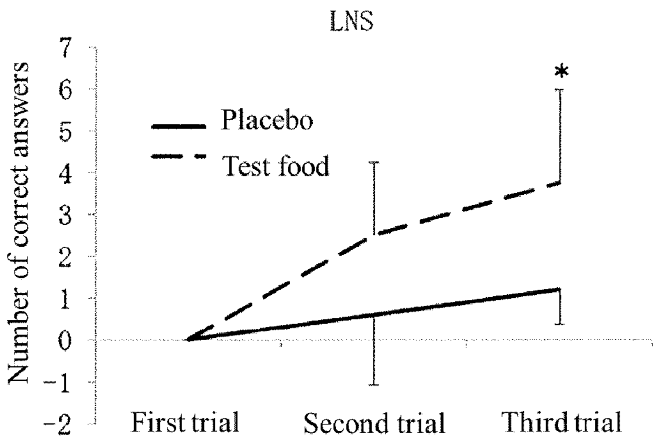


FIGURE 9

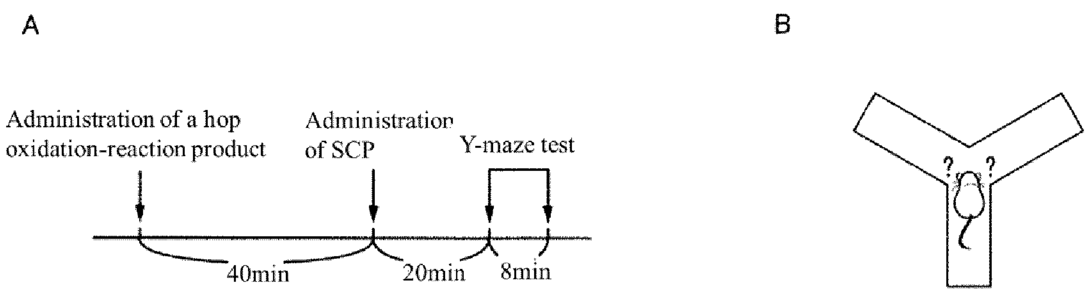


FIGURE 10

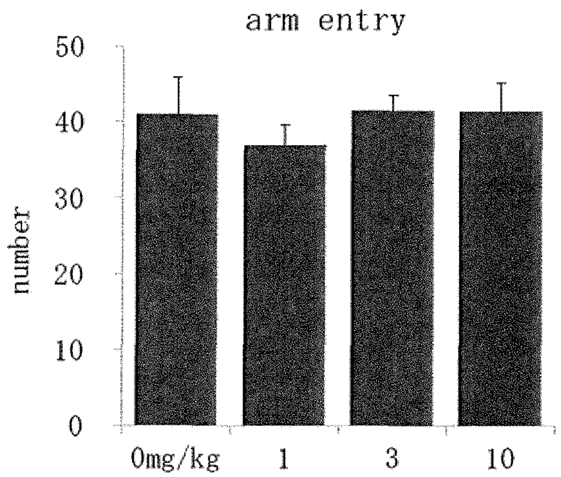


FIGURE 11

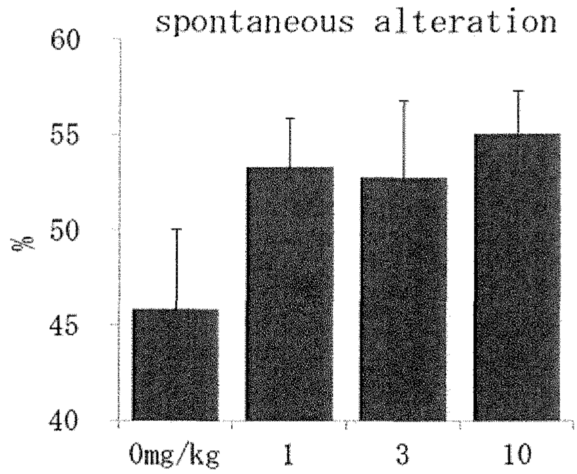


FIGURE 12

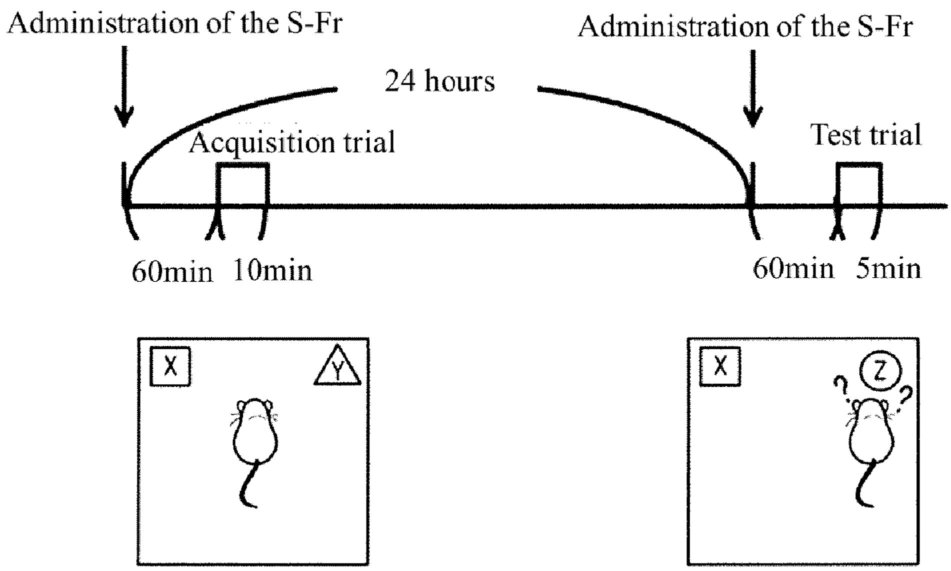


FIGURE 13

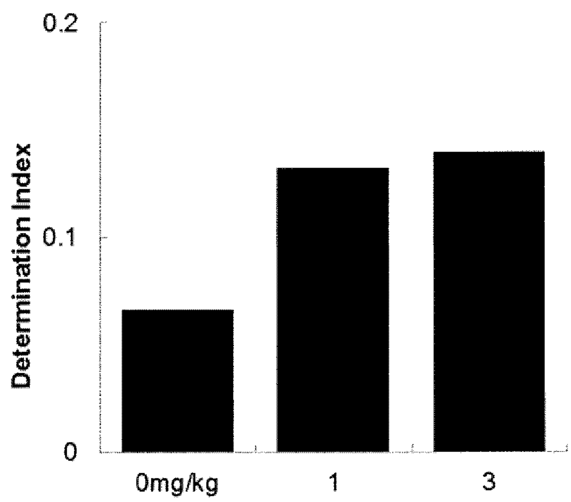


FIGURE 14

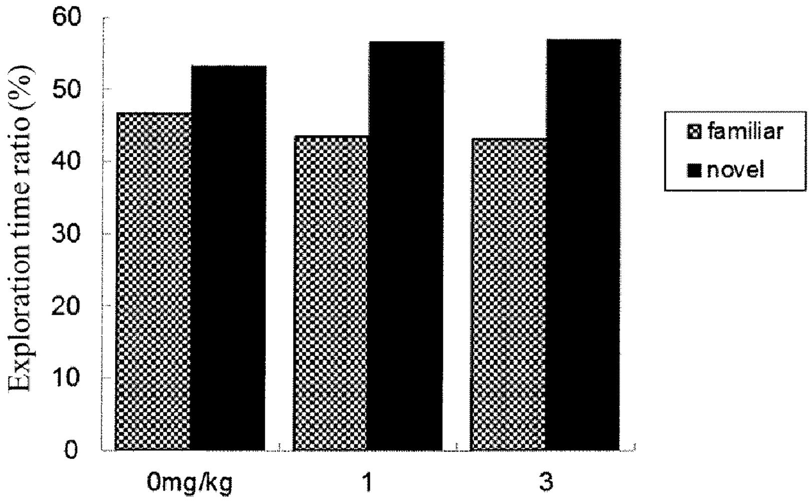


FIGURE 15

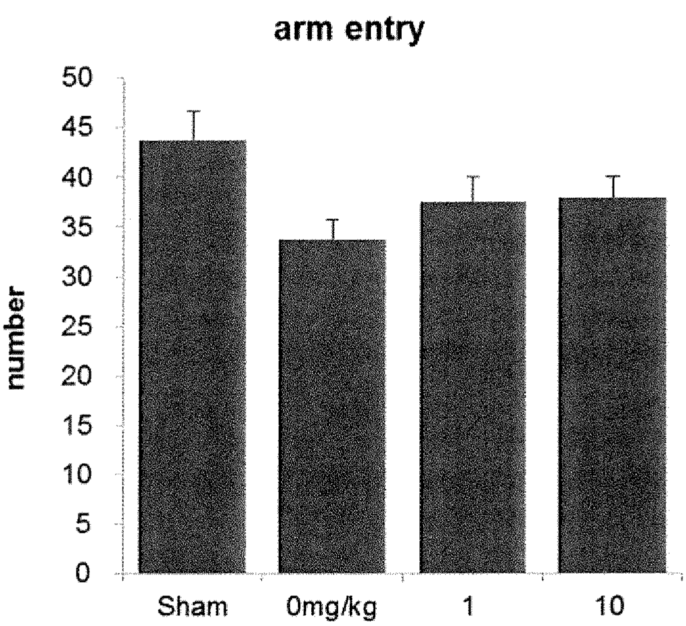


FIGURE 16

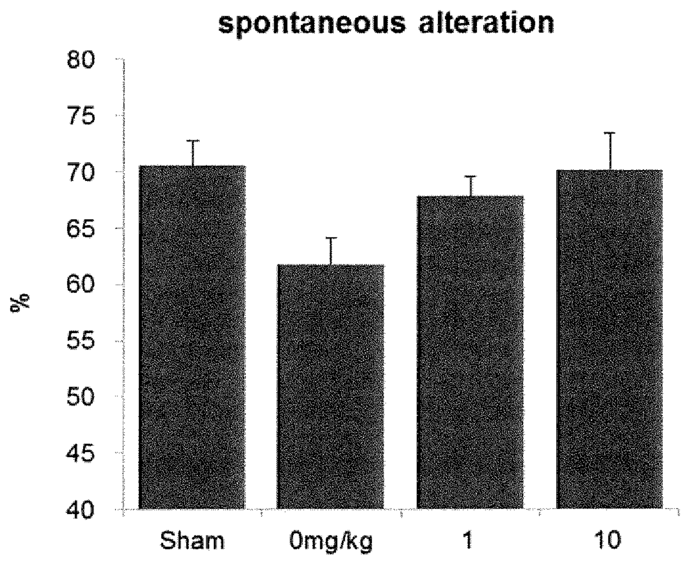


FIGURE 17

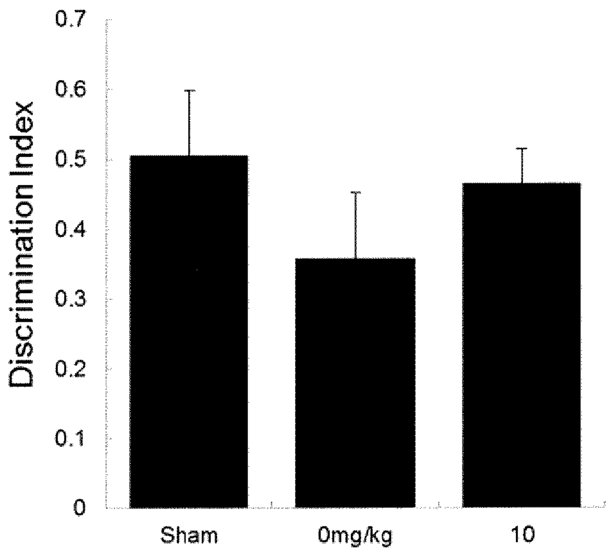


FIGURE 18

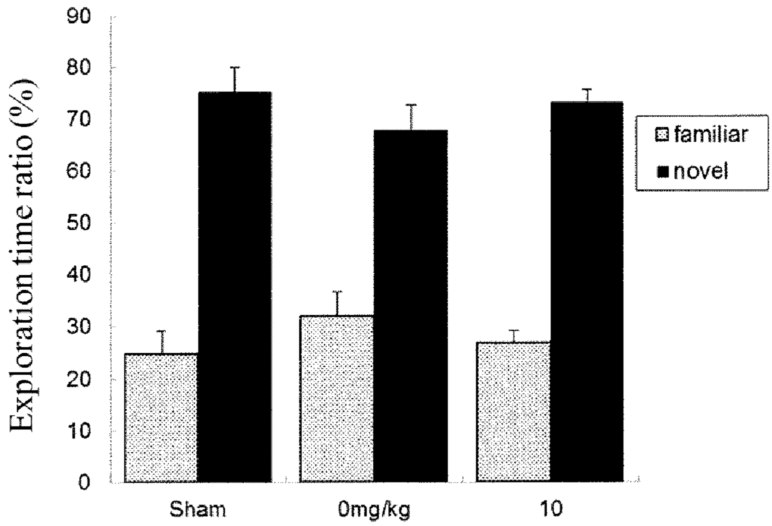
Exploration time ratio (%)
90
80
70
60
50
40
30
20
10
0
Sham
0mg/kg
10
familiar
novel

FIGURE 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016079304 A
- WO 2012081675 A


### Non-patent literature cited in the description


- **TOSHIHISA TANAKA** ; **MASATOSHI TAKEDA.** *Nippon-Rinsho*, 2011, 52-56
- *Biosci. Biotechnol. Biochem.*, 2015, vol. 79, 1684-1694
- *Biosci. Biotechnol. Biochem.*, 2015 (79), 1684-1694
- *J. Agric. Food Chem.*, 2015, vol. 63, 10181-10191
- *J. Nat. Prod.*, 2014, vol. 77, 1252-1261
- **NOBUHISA IWATA** ; **TAKAOMI SAIDO**. Unraveling the Mystery of Alzheimer's disease. *Chugai-Igakusha*, 2010, 173-180